# EUROPÄISCHE PATENTSCHRIFT

(11) **EP 1 158 915 B1**
(45) Veröffentlichungstag und Bekanntmachung des Hinweises auf die Patenterteilung: **01.09.2004**
(21) Anmeldenummer: 99906009.8
(22) Anmeldetag: 09.03.1999
(51) Int. Cl.: A61B 17/80

(54) **KNOCHENPLATTE**
BONE PLATE
PLAQUE POUR OSTEOSYNTHESE

(43) Veröffentlichungstag der Anmeldung: 05.12.2001
(73) Patentinhaber: SYNTHES AG Chur, 7002 Chur (CH)
(72) Erfinder: WAGNER, Michael, A-1190 Wien (AT); FRIGG, Robert, CH-2544 Bettlach (CH); SCHAVAN, Robert, D-47877 Willich Anrath (DE)
(74) Vertreter: Lusuardi, Werther Giovanni, Dr.
(86) Internationale Anmeldenummer: PCT/CH1999/000106
(87) Internationale Veröffentlichungsnummer: WO 2000/053110

(56) Entgegenhaltungen:
- WO-A-97/09000
- DE-A- 4 341 980
- DE-A- 4 343 117
- US-A- 4 408 601
- US-A- 4 927 421
- US-A- 5 002 544
- US-A- 5 709 686

## Beschreibung

Die Erfindung betrifft eine Knochenplatte gemäss dem Oberbegriff des Patentanspruchs 1 sowie eine Fixationsvorrichtung mit einer solchen Knochenplatte gemäss dem Oberbegriff des Patentanspruchs 14.

Grundsätzlich kennt man zwei Arten der mit Knochenplatten erfolgenden Osteosynthese.

Die erste betrifft die "Rigide Osteosynthese". Die rigide Osteosynthese wird bei der Versorgung von Gelenksfrakturen, einfachen Schaftfrakturen (wenn keine Nagelung vorgenommen werden kann) sowie bei Osteotomien angewandt. Neben der Anatomischen Repositionsmöglichkeit unterstützt der Knochen selber die Stabilität der Osteosynthese, was zu einer früheren und schmerzfreieren Belastung der Extremität führt. Vorteile einer stabilen Frakturversorgung können auch dort beobachtet werden, wo die Knochendurchblutung durch das Trauma beding stark vermindert ist. Bei der Versorgung von "non-unions" oder bei vorhandener Infektion, muss die Fraktur stabil versorgt werden, um eine Knochenheilung zu ermöglichen und um die Infektion nicht durch die Instabilität im Frakturspalt zusätzlich zu reizen.

Die zweite betrifft die "Flexible Osteosynthese". Die grössten Vorteile der flexiblen (biologischen) Osteosynthese sind bei der Versorgung von Trümmerfrakturen im Schaftbereich von Röhrenknochen zu sehen. Bei diesen Frakturen ist das Ziel die Länge des Knochens, sowie die Knochenenden (Gelenke) in korrekter Lage zueinander zu halten. Die Frakturzone wird dabei nicht direkt fixiert oder manipuliert, was die Durchblutung dieser Zone nicht zusätzlich belastet. Die Knochenplatten funktionieren ähnlich einem Verriegelungs-Marknagel, der nur in den Metaphysen verankert ist.

Betrachtet man nun diese beiden Extreme der Plattenosteosynthese, erkennt man wie weit diese auseinander liegen. Da sich nicht immer alle Frakturen in eine der beiden oben genannten Osteosynthese-Arten einteilen lassen, muss der Chirurg oft Kompromisse eingehen, da ihm kein Implantat zur Verfügung steht, welches ihm erlaubt beide Methoden kompromisslos zu kombinieren. Eine solche Kombination wäre z.B. dann sinnvoll, wenn eine Gelenksfraktur mit Zugschrauben durch die Knochenplatte komprimiert werden kann und der gesamte Gelenksteil über einen internen Fixateur, mit winkelstabilen Schrauben, zur Diaphyse verbunden wird. Ein weiter Anwendungsfall wäre z.B. bei porotischem Knochen , wo eine Knochenplatte mit axial und winkelstabilen Schrauben im metaphysären Fragment verankert werden kann, wobei im diaphysären Bereich eine stabile Verplattung vorgenommen werden kann, mit der Unterstützung einer Plattenzugschraube durch die Fraktur. Dank dieser Versorgung kann eine primäre Frakturstabilisierung erreicht werden.

Diese Situation hat dazu geführt, dass man Knochenimplantate für beide Arten der Osteosynthese entwickelt und auf den Markt. gebracht hat. Beide Implantategruppen sind für ihre jeweilige Methode optimal ausgelegt. Der Nachteil dieser beiden System liegt somit in ihrer fehlenden Kombinationsmöglichkeit.

Aus der US 5,709,686 TALOS ET AL. ist eine derartige Kombinationsplatte bekannt, bei welcher ein zylindrisches Gewinde in der mittleren Partie des Langlochs angebracht ist. Die Nachteile dieser bekannten Platte sind die folgenden:
1) Das mittständig im Langloch der Platte angeordnete Gewinde ist auf einen Bereich zwischen 60° bis 179° beschränkt
2) Die mittständige Lage des Gewindes mit einem Bereich zwischen 60° bis 179°im Langloch (Spannloch) der Platte weist die Gefahr auf, dass sich die seitlichen Stege des Langlochs aufweiten können.
3) Wegen der zylindrischen Form des Gewindes muss ein speziell ausgebildeter Schraubenkopf verwendet werden, der sich beim Eindrehen auf der Plattenoberfläche abstützen kann.

Hier will die Erfindung Abhilfe schaffen. Der Erfindung liegt das Problem zugrunde, eine Knochenplatte zu schaffen, welche beide Osteosynthesearten in sich kombiniert, ohne jedoch Einschränkung bei den beiden reinen Plattenversorgungsmethoden zur Folge zu haben. Sie soll demnach die kompromisslose Verwendung der Platte als Kompressionsplatte und als sogenannter Fixateur interne erlauben.

Die Erfindung löst die gestellte Aufgabe mit einer Knochenplatte, welche die Merkmale des Anspruchs 1 aufweist.

Die Anwendung der Platte als Fixateur interne führt zu einer stark erhöhten mechanischen Beanspruchung des Platten-Schrauben-Interface, da die Platte nicht auf den Knochen gedrückt wird und so die Knochenfraktur mittels Reibung zwischen Platte und Knochen fixiert wird. Dieser mechanischen Mehrbelastung wird dadurch Rechnung getragen, dass sich das Gewinde im Langloch über einen Bereich von mindestens 180° erstreckt und somit das Schraubenkopf-Gewinde um mindestens diesen Winkelbereich umschliesst. Bei dünnen Knochenplatten ist dieser Umstand von besonderer Bedeutung.

Eine bevorzugte Weiterbildung besteht darin, dass das sich das Innengewinde des Langlochs gegen die Unterseite der Knochenplatte hin konisch verjüngt. Sie hat den Vorteil, dass die Fixation der Schraube durch das konische Gewinde des Plattenlochs und das korrespondierende konische Gewinde des verwendeten Schraubenkopfes erfolgt. Diese Art der Fixation ist besonders wichtig, wenn man selbstbohrende Schrauben verwenden will. Dank des konischen Gewindes im Kopfbereich der Schraube, kann der Einbringvorgang der Schraube in den Knochen, unabhängig von der Platte erfolgen. Erst wenn der Gewindekonus des Schraubenkopfes in das Innengewinde des Langlochs der Platte eindringt, wird die Schraube blockiert. Trotz unterschiedlicher Gewindeanfänge im Plattenloch-Konus und im Knochen zentriert sich das konische Schraubenkopfgewinde im Gewindekonus der Platte.
Beim Festziehen des konischen Gewindes entstehen radiale Kräfte im Plattenloch. Um diese ausreichend aufzunehmen, muss das konische Plattenloch eine ausreichende Stabilität aufweisen. Das gegen die Unterseite der Knochenplatte hin sich konisch verjüngende Innengewinde weist zweckmässigerweise einen Konuswinkel von 5 - 20° auf, typischerweise von 10° auf.

Bei einer weiter bevorzugten Ausführungsform sind die Löcher gemäss den Merkmalen A und B miteinander identisch, so dass das Innengewinde innerhalb eine Loches angebracht ist, dessen Durchmesser D_{L} in Richtung der Plattenlängsachse gemessen grösser ist als der Durchmesser D_{Q} dieses Loches senkrecht zur Plattenlängsachse gemessen.
Bei einer weiteren bevorzugten Ausbildung der Erfindung erstreckt sich das Innengewinde über einen Bereich von 200° bis 250° des von ihm gebildeten geometrischen Körpers.

Falls das Langlochs konisch ausgebildet ist ergibt die Messung der Ausdehnung des Innengewindes an der Unterseite, bzw. an der Oberseite der Platte verschieden grosse Werte. Bei einer Messung an der Unterseite sollte sich der Bereich des Gewindes vorzugsweise über 180° bis 230° erstrecken; bei einer Messung an der Oberseite über 200° bis 270°.

Bei einer weiteren bevorzugten Ausführungsform ist das endständige, konische Gewinde im Langloch (Spannloch) an jenem Ende angebracht, welches näher zur Plattenmitte liegt. Dies hat den Vorteil, dass die Spannfunktion der Plattenspannlöcher nicht beeinträchtigt wird.

Bei einer weiteren bevorzugten Ausführungsform weist mindestens eines der Löcher mit Innengewinde in seinem oberen, der Oberseite zugewandten Teil, eine konkave, vorzugsweise sphärische. Erweiterung zur Aufnahme einer Knochenschraube mit einem kugeligen Kopf auf. Der kugelförmige Schraubenkopf einer herkömmlichen Knochenschraube findet in dieser konkaven, sphärischen Erweiterung einen optimalen Sitz. Dies vor allem dann, wenn die Knochenschraube exzentrisch eingebracht wurde, was zur Erreichung einer Frakturkompression nötig ist.

Bei einer weiteren bevorzugten Ausführungsform ist die Unterseite konkav ausgebildet ist. Durch die konkave Unterseite der Platte, passt sich diese besser an den runden Knochenquerschnitt der Tibia, des Femurs, des Humerus und der Unterarmknochen an. Durch die konkave Ausführungsform der Plattenunterseite, kann eine herkömmliche Knochenschraube schräg durch das Platteloch eingesetzt werden. Das kann vor allem für das Fassen eines kleinen Knochenfragments wichtig sein, das an die Platte herangezogen werden muss.

Bei einer weiteren bevorzugten Ausführungsform erstreckt sich das Innengewinde über die gesamte Höhe der Knochenplatte von der Unterseite bis zur Oberseite, um einen möglichst hohe Stabilität zu erreichen.

Bei einer weiteren bevorzugten Ausführungsform erweitert sich das Langloch im seinem gewindefreien Sektor, in seinem unteren, der Unterseite zugewandten Teil, so dass eine Auslenkung der Knochenschraube möglich wird.

Bei einer weiteren bevorzugten Ausführungsform liegt das Verhältnis zwischen D_{L}/D_{Q} im Bereich von 1,01 - 3,00, vorzugsweise im Bereich von 1,1 - 1,5. Dieses Verhältnis ergibt sich aus der Kombination des Kompressionsloches - das einen gewissen Spannweg für die Schraube benötigt- und des Gewindeloches. Das ermittelte Verhältnis D_{L}/D_{Q} stellt einen optimalen Kompromiss zwischen der Spannmöglichkeit und der minimalen Plattenschwächung durch das Kombinationsloch dar.

Eine weitere Ausführungsform umfasst neben der erfindungsgemässen Knochenplatte zusätzlich mindestens eine Knochenschraube mit einem zum Innengewinde korrespondierenden, am Schraubenkopf angebrachten Aussengewinde, welche vorzugsweise selbstbohrend ausgebildet ist.
Bei der Verwendung der Knochenplatte als Kompressionsplatte, wird die Spannlochgeometrie der Plattenbohrung, durch das endständige, konische Gewindeloch 4, nicht negativ beeinflusst. Der Vorteil der konischen Ausführung des Gewindeloches ist das plattenunabhängige Einbringen der Schraube in den Knochen, wobei sich die Schraube erst beim Festziehen mit der Platte, über einen entsprechend konisch ausgebildeten, gewindeten Schraubenkopf, verbindet. Das ist vor allem bei der Verwendung von selbstbohrenden, selbstschneidenden Schrauben vorteilhaft.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine Aufsicht auf die erfindungsgemässe Knöchenplatte mit einem Langloch mit integriertem Gewinde;
Fig. 2 einen Längsschnitt durch das Langloch mit Gewinde von Fig. 1; und
Fig. 3 eine perspektivische Darstellung durch die erfindungsgemässe Knochenplatte mit einer im Langloch mit integriertem Gewinde eingesetzten Knochenschraube.

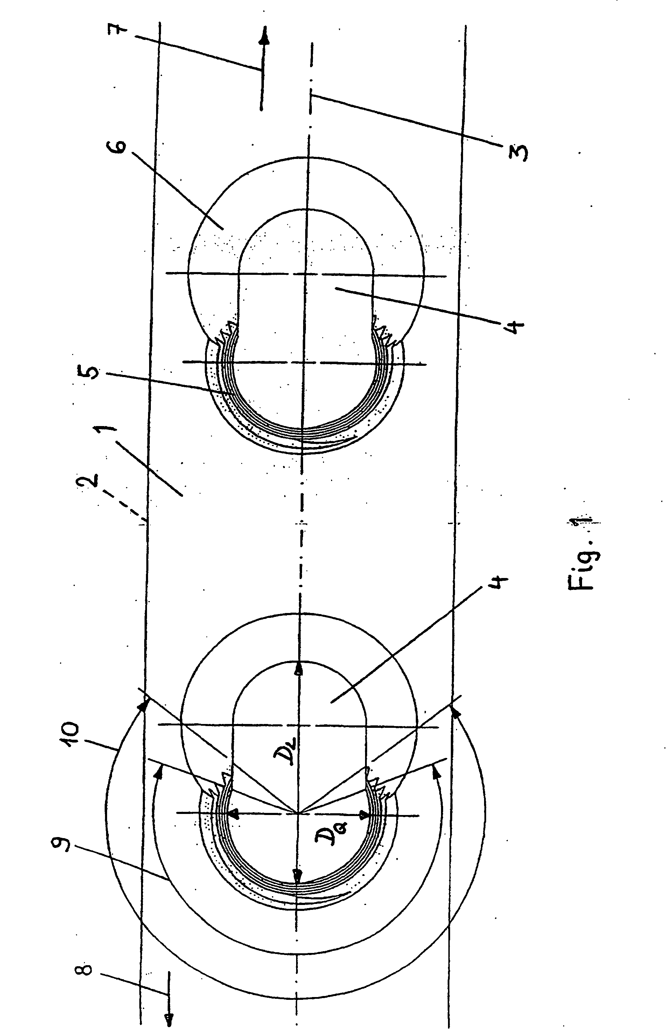

Die in Fig. 1 dargestellte erfindungsgemässe Knochenplatte besitzt eine Oberseite 1, eine für den Knochenkontakt bestimmte Unterseite 2 sowie zwei die Oberseite 1 mit der Unterseite 2 verbindenden, entlang der Plattenlängsachse 3 angeordneten Löchern 4 für die Aufnahme von Knochenschrauben.

Der Pfeil 7 zeigt die Richtung zum einem Ende der Knochenplatte währenddem der Pfeil 8 die Richtung zur Plattenmitte anzeigt.

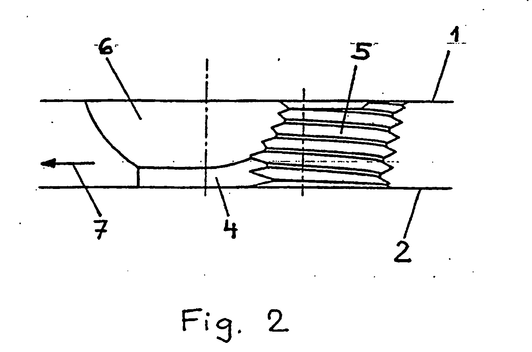

Der Durchmesser D_{L} des näher zur Plattenmitte gelegenen Lochs 4 ist in Richtung der Plattenlängsachse 3 gemessen grösser als der Durchmesser D_{Q} dieses Loches senkrecht zur Plattenlängsachse 3 gemessen. Der Durchmesser D_{L} beträgt 5,2 mm und der Durchmesser D_{Q} 3 mm.
Wie in Fig. 2 dargestellt weist dieses Langloch in seinem oberen, der Oberseite 1 zugewandten Teil, eine konkave, vorzugsweise sphärische Erweiterung 6 zur Aufnahme einer Knochenschraube mit, einem kugeligen Kopf auf. Das Plattenloch hat im Bereich des endständig darin angeordneten Innengewindes die Form eines sich gegen die Unterseite 2 hin verjüngenden Konus, so dass sich das Innengewinde 5 ebenfalls gegen die Unterseite 2 der Knochenplatte hin konisch verjüngt und zwar mit einem Konuswinkel von 10°. Das Innengewinde 5 ist vorzugsweise als doppelgängiges Gewindes augebildet. Das Innengewinde 5 erstreckt sich dabei über die gesamte Höhe der Knochenplatte von der Oberseite 1 bis zur Unterseite 2.
Wie in Fig. 1 dargestellt erstreckt sich das Innengewinde 5 an der Unterseite 2 der Knochenplatte gemessen - wie durch den Kreisbogen 9 angedeutet - über einen Bereich von 223° und an der Oberseite 1 gemessen - wie durch den Kreisbogen 10 angedeutet - über einen Bereich von 256°.

Je nach Durchmesser des Innengewindes 5 ergeben sich folgende bevorzugte Parameter:

| Durchmesser des Gewindes | 3,0 mm | 4,0 mm | 5,0 mm |
|---|---|---|---|
| zweigängiges Gewinde | JA | JA | JA |
| Steigung des Gewindes | 0,7 0,9 | | 1,0 |
| Tiefe des Gewindes | 0,2025 | 0,2575 | 0,2810 |
| (= halbe Differenz zwischen Aussen- und Innendurchmesser) | | | |
| Winkelbereich (an Oberseite) | 200° | 200° | 190° |
| Winkelbereich (an Unterseite) | 260° | 240° | 250° |

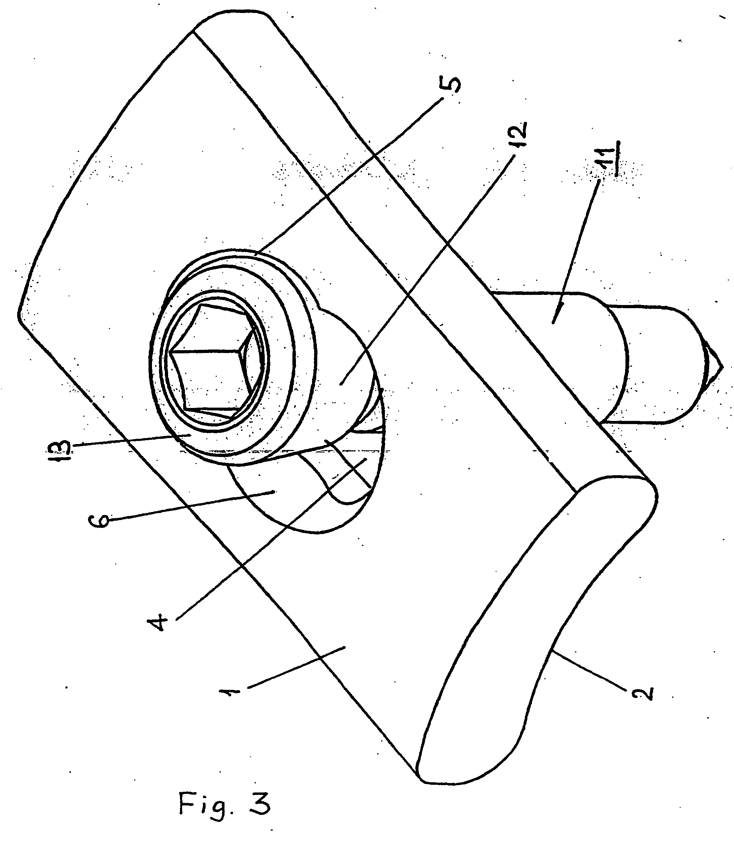

In Fig. 3 ist eine Fixationsvorrichtung mit einer Knochenplatte gemäss den Fig. 1 und 2 dargestellt, bei der eine Knochenschraube 11 mit einem zum Innengewinde 5 der Knochenplatte korrespondierenden, am Schraubenkopf 13 angebrachten Aussengewinde 12 umfasst. Die Knochenschraube 11 ist zweckmässigerweise selbstbohrend und selbstschneidend ausgebildet.

## Patentansprüche

1. Knochenplatte mit einer Oberseite (1), einer für den Knöchenkontakt bestimmten Unterseite (2) sowie mehreren die Ober- mit der Unterseite (1;2) verbindenden, entlang der Plattenlängsächse (3) angeordneten Löchern (4) für die Aufnahme von Knochenschrauben (11),
**dadurch gekennzeichnet, dass**
der Durchmesser D_{L} mindestens eines der Löcher (4) in Richtung der Plattenlängsachse (3) gemessen grösser ist als der Durchmesser D_{Q} dieses Langloches senkrecht zur Plattenlängsachse (3) gemessen, und dass das Langloch ein Innengewinde (5) aufweist, welches sich über 190° bis 280° des von ihm gebildeten geometrischen Korpers erstreckt.

2. Knochenplatte nach Anspruch 1, **dadurch gekennzeichnet, dass** sich das Innengewinde (5) gegen die Unterseite (2) der Knochenplatte hin konisch verjüngt.

3. Knochenplatte nach Anspruch 1 oder 2, **dadurch gekennzeichnet, dass** sich das Innengewinde (5) über 200° bis 250° des von ihm gebildeten geometrischen Körpers erstreckt.

4. Knochenplatte nach einem der Ansprüche 1 bis 3, **dadurch gekennzeichnet, dass** sich das Innengewinde (5) - an der Unterseite (2) gemessen - über 190° bis 230° erstreckt und - an der Oberseite (1) gemessen - über 200° bis 270° erstreckt.

5. Knochenplatte nach einem der Ansprüche 1 bis 4, **dadurch gekennzeichnet, dass** das Innengewinde (5) - in Richtung der Plattenlängsachse (3) gesehen - an einem der beiden Enden des Langlochs, vorzugsweise näher zur Plattenmitte (8) angebracht ist.

6. Knochenplatte nach einem der Ansprüche 1 bis 5, **dadurch gekennzeichnet, dass** mindestens eines der Langlöcher in seinem oberen, der Oberseite (1) zugewandten Teil, eine konkave Erweiterung (6) zur Aufnahme einer Knochenschraube (11) mit einem kugeligen Kopf (13) aufweist.

7. Knochenplatte nach Anspruch 6, **dadurch gekennzeichnet, dass** die konkave Erweiterung (6) sphärisch ausgebildet ist.

8. Knochenplatte nach einem der Ansprüche 1 bis 7, **dadurch gekennzeichnet, dass** die Unterseite (1) konkav ausgebildet ist.

9. Knochenplatte nach einem der Ansprüche 1 bis 8, **dadurch gekennzeichnet, dass** sich das Innegewinde (5) über die gesamte Höhe der Knochenplatte von der Oberseite (1) bis zur Unterseite (2) erstreckt.

10. Knochenplatte nach einem der Ansprüche 1 bis 9, **dadurch gekennzeichnet, dass** sich das Langloch im seinem gewindefreien Sektor, in seinem unteren, der Unterseite (2) zugewandten Teil erweitert.

11. Knochenplatte nach einem der Ansprüche 1 bis 10, **dadurch gekennzeichnet, dass** das Verhältnis zwischen D_{L}/D_{Q} im Bereich von 1,01 - 3,00 liegt.

12. Knochenplatte nach Anspruch 11, **dadurch gekennzeichnet, dass** das Verhältnis zwischen D_{L}/D_{Q} im Bereich von 1,1 - 1,5 liegt.

13. Knochenplatte nach einem der Ansprüche 2 bis 12, **dadurch gekennzeichnet, dass** das gegen die Unterseite (2) der Knochenplatte hin sich konisch verjüngende Innengewinde (5) einen Konuswinkel von 5 - 20° aufweist.

14. Fixationsvorichtung mit einer Knochenplatte gemäss einem der Ansprüche 1 bis 13, **dadurch gekennzeichnet, dass** sie zusätzlich mindestens eine Knochenschraube (11) mit einem zum Innengewinde (5) korrespondierenden, am Schraubenkopf (13) angebrachten Aussengewinde (12) umfasst.

15. Fixationsvorrichtung nach Anspruch 14, **dadurch gekennzeichnet, dass** die Knochenschraube (11) selbstbohrend ausgebildet ist.

16. Fixationsvorrichtung nach Anspruch 14 oder 15, **dadurch gekennzeichnet, dass** die Knochenschraube (11) selbstschneidend ausgebildet ist.

## Claims

1. A bone plate comprising a top side (1), an underside (2) that shall make contact with the bone, and several holes (4) from the top side (1) to the underside (2) and configured along the longitudinal plate axis (3) and receiving bone screws (11),
**characterized in that**
the diameter D_{L} of at least one of these holes (4) when measured in the direction of the longitudinal plate axis (3) is larger than the diameter D_{Q} of this elongated hole perpendicularly to the longitudinal plate axis (3), and is fitted with an inside thread (5), which subtends an arc of 190° to 280° of the geometric body defined by it.

2. Bone plate as claimed in claim 1, **characterized in that** the inside thread (5) tapers conically in the direction of the underside (2) of the bone plate.

3. Bone plate as claimed in claim 1 or 2, **characterized in that** the inside thread (5) subtends an arc at least of 200 to 250° of the geometric body defined by it.

4. Bone plate as claimed in one of claims 1 through 3, **characterized in that** when measured at the underside (2) the inside thread runs across an arc of 190° to 230° and when measured at the top side (1) it runs across an arc of 200 to 270°.

5. Bone plate as claimed in one of the claims 1 through 4, **characterized in that** when seen in the direction of the longitudinal plate axis (3), the inside thread (5) is situated at one of the two ends of the elongated hole, preferably nearer the plate's center (8).

6. Bone plate as claimed in one of claims 1 through 5, **characterized in that** at least one of the elongated hole comprises a concave recess (6) in its portion facing the top side (1) to receive a bone screw (11) having a spherical head (13).

7. Bone plate as claimed in claim 6, **characterized in that** the concave recess (6) is spherically shaped.

8. Bone plate as claimed in one of claims 1 through 7, **characterized in that** the underside (1) is concave.

9. Bone plate as in one of claims 1 through 8, **characterized in that** the inside thread (5) runs through the full thickness of the bone plate from the top side (1) to the underside (2).

10. Bone plate as claimed in one of claims 1 through 9, **characterized in that** the hole (4) flares in its unthreaded sector in its lower portion facing the underside (2).

11. Bone plate as claimed in one of claims 1 through 10, **characterized in that** the ratio of D_{L}/D_{Q} is in the range of 1.01 to 3.00.

12. Bone plate as claimed in claim 11, **characterized in that** the ratio of D_{L}/D_{Q} is in the range of 1.1 to 1.5.

13. Bone plate as claimed in one of claims 2 through 12, **characterized in that** the cone angle of the inside thread (5) conically tapering toward the bone plate's underside is between 5 and 20°.

14. An affixation system comprising a bone plate claimed in one of claims 1 through 13, **characterized in that** in addition it comprises at least one bone screw (11) fitted with an outer thread (12) at the screwhead (13), said outer thread (12) matching the inside thread (5).

15. Affixation system as claimed in claim 14, **characterized in that** the bone screw (11) is self-drilling.

16. Affixation system as claimed in either of claims 14 and 15, **characterized in that** the bone screw (11) is self-tapping

## Revendications

1. Plaque d'ostéosynthèse comprenant une face supérieure (1), une face inférieure (2) destinée à être en contact avec l'os ainsi que plusieurs trous (4) reliant la face supérieure (1) et la face inférieure (2), disposés le long de l'axe longitudinal (3) de la plaque et destinés à recevoir des vis d'ostéosynthèse (11),
**caractérisée en ce que**
le diamètre D_{L} d'au moins un des trous (4), mesuré dans le sens de l'axe longitudinal (3) de la plaque, est supérieur au diamètre D_{Q} de ce trou oblong, mesuré perpendiculairement à l'axe longitudinal (3) de la plaque, et **en ce que** le trou oblong présente un filet intérieur (5) qui s'étend sur 190° à 280° du corps géométrique formé par lui.

2. Plaque d'ostéosynthèse selon la revendication 1, **caractérisée en ce que** le filet intérieur (5) va en s'amincissant de manière conique vers la face inférieure (2) de la plaque d'ostéosynthèse.

3. Plaque d'ostéosynthèse selon la revendication 1 ou 2, **caractérisée en ce que** le filet intérieur (5) s'étend sur 200° à 250° du corps géométrique formé par lui.

4. Plaque d'ostéosynthèse selon les revendications 1 à 3, **caractérisée en ce que** le filet intérieur (5) - mesuré au niveau de la face inférieure (2) - s'étend sur 190° à 230° et - mesuré au niveau de la face supérieure (1) - s'étend sur 200° à 270°.

5. Plaque d'ostéosynthèse selon l'une des revendications 1 à 4, **caractérisée en ce que** le filet intérieur (5) - vu dans le sens de l'axe longitudinal (3) de la plaque - est ménagé à l'une des deux extrémités du trou oblong, de préférence à celle située plus près du centre (8) de la plaque.

6. Plaque d'ostéosynthèse selon l'une des revendications 1 à 5, **caractérisée en ce qu'**au moins un des trous oblongs présente, dans sa partie supérieure tournée vers la face supérieure (1), un élargissement concave (6) destiné à recevoir une vis d'ostéosynthèse (11) comprenant une tête sphérique (13).

7. Plaque d'ostéosynthèse selon la revendication 6, **caractérisée en ce que** ledit élargissement concave (6) est réalisé de manière sphérique.

8. Plaque d'ostéosynthèse selon l'une des revendications 1 à 7, **caractérisée en ce que** la face inférieure (1) est réalisée de manière concave.

9. Plaque d'ostéosynthèse selon l'une des revendications 1 à 8, **caractérisée en ce que** le filet intérieur (5) s'étend sur l'ensemble de la hauteur de la plaque d'ostéosynthèse, à savoir de la face supérieure (1) jusqu'à la face inférieure (2).

10. Plaque d'ostéosynthèse selon l'une des revendications 1 à 9, **caractérisée en ce que** le trou oblong s'élargit dans sa partie dépourvue de filet, à savoir dans sa partie inférieure tournée vers la face inférieure (2).

11. Plaque d'ostéosynthèse selon l'une des revendications 1 à 10, **caractérisée en ce que** le rapport D_{L}/D_{Q} se situe dans une zone comprise entre 1,01 et 3,00.

12. Plaque d'ostéosynthèse selon la revendication 11, **caractérisée en ce que** le rapport D_{L}/D_{Q} se situe dans une zone comprise entre 1,1 et 1,5.

13. Plaque d'ostéosynthèse selon l'une des revendications 2 à 12, **caractérisée en ce que** le filet intérieur (5) qui va en s'amincissant de manière conique vers la face inférieure (2) de la plaque d'ostéosynthèse présente un angle de cône compris entre 5° et 20°.

14. Dispositif de fixation comprenant une plaque d'ostéosynthèse selon l'une des revendications 1 à 13, **caractérisé en ce qu'**il comprend, en outre, au moins une vis d'ostéosynthèse (11) comprenant un filet extérieur (12) ménagé dans la tête de vis (13) et correspondant au filet intérieur (5).

15. Dispositif de fixation selon la revendication 14, **caractérisé en ce que** la vis d'ostéosynthèse (11) est réalisée sous forme de vis autoforeuse.

16. Dispositif de fixation selon la revendication 14 ou 15, **caractérisé en ce que** la vis d'ostéosynthèse (11) est réalisée comme sous forme de vis autotaraudeuse.
